# EUROPEAN PATENT APPLICATION

(11) **EP 3 757 221 A1**
(43) Date of publication of application: **30.12.2020**
(21) Application number: 19182823.5
(22) Date of filing: 27.06.2019
(51) Int. Cl.: C12P 7/64, C12M 1/00, C12M 1/12

(54) **METHOD FOR PRODUCING POLYUNSATURATED FATTY ACIDS USING MICROALGAE**

(71) Applicant: Phytolinc UG, 50937 Köln (DE)
(72) Inventor: Zaabe, Jan-Nicolas, 50939 Köln (DE); Maercker, Arne, 50823 Köln (DE); Prausse, Dennis, 50935 Köln (DE)
(74) Representative: Remus, Alvaro Johannes

(57) **Abstract**

The invention relates to a method for producing at least one polyunsaturated fatty acid using microalgae, wherein the microalgae are cultivated by exposing the microalgae to light and providing them with at least one nutrient. According to the invention the microalgae are immobilized on at least one porous substrate 20 and their cultivation is employed on the substrate 20. As an initial step, before the microalgae are cultivated for producing the polyunsaturated fatty acids, the microalgae can be expanded for maximizing biomass. For example, the polyunsaturated fatty acid may be at least one of eicosapentaenoic acid and docosahexaenoic acid.

## Description

### Background of the invention

The invention relates to a method for producing at least one polyunsaturated fatty acid (PUFA) using microalgae, wherein the microalgae are cultivated by exposing the microalgae to light and providing them with at least one nutrient.

### Prior art

Living phototrophic organisms such as microalgae include a high amount of protein and vitamin B12 and contain valuable unsaturated fatty acids as well as natural pigments. Therefore, they serve as raw material in the food supplement and cosmetics industry and are cultivated by microalgae producers. Commercial producers usually cultivate microalgae in open ponds, tubular or plate-shaped photobioreactors. The underlying cultivation technique for all commercially available photobioreactors is suspension culture in which the algae cells are cultivated free-swimming in a water column. High costs for the concentration of algae biomass from the medium suspension as well as costly mechanical harvesting and the following drying of the algae cells are drawbacks of suspension culture based systems. Also, the increasing cell density makes an optimal lighting of the cells inside the suspension culture more difficult.

Polyunsaturated fatty acids (PUFA), such as eicosapentaenoic acid (EPA, C20:5 Omega-3) and docosahexaenoic acid (DHA, C22:6 Omega-3) are of high commercial interest. They are used as food supplement, feed supplement or in therapeutic applications. Currently, these unsaturated fatty acids are mainly gained from fish oil. At the moment the amount of wild caught fish is decreasing, while the demand for unsaturated fatty acids for food or feed supplement products is increasing. Therefore, in order to secure the supply of unsaturated fatty acids in a world with a steadily growing population, new production sources are becoming increasingly important.

As part of the food chain, microalgae are placed quite low but are part of an important basal step. They produce unsaturated fatty acids which subsequently become enriched in the higher trophic levels. So far, only a few species are used for the commercial production of unsaturated fatty acids. Most of them belong to the genera *Schizochytrium sp.* (Labyrinthulea, Thraustochytrida); *Crypthecodinium sp.* (Dinophyceae, Peridiniales); *Phaeodactylum sp.* (Bacilariophyceae, Naviculales) and *Odontella sp.* (Bacilariophyceae, Triceratiales). For example, microalgae of the genera Odontella are cosmopolitan and are usually the size of 35-50 µm. The frustule exists of two bowl-shaped parts, Epitheka and Hypotheka. Naturally, algae dry mass of *Odontella aurita* has an EPA concentration between 1,6 and 3,4%. Thereby, the share of EPA in relation to the total fatty acids composition equals approx. 20%. *Odontella aurita* is mainly produced autotrophically in open ponds or, less often, in closed photobioreactors (Pulz et Gross (2004): Valuable products from biotechnology of microalgae. Appl. Microbiol. Biotechnol. 65(6):635-648).

US 2014/0120590 A1 discloses a mixotrophic as well as heterotrophic production process. In the heterotrophic or mixotrophic cultivation process, a carbon source such as starch, glucose, saccharose, xylose, arabinose, lactose, lactates, cellulose or acetate is added to the culture medium. Thereby, the concentration of the carbon source in the culture medium is between 10 and 200 mM per liter. For *Odontella aurita* it is known that triggering a stress reaction increases the EPA-share of the total fatty acids composition. In US 2014/0120590 A1, during the mixotrophic cultivation process, the reactor is constantly lit up with flash lights. This leads to a stress reaction of the algae and increases the share of unsaturated fatty acids in the algae cells. The described production process leads to a 20-40% increase of the biomass production as well as an increase in the EPA- and DHA-concentration relative to total lipids to 10-50%.

As described in US 2014/0120590 A1, a bioreactor with suspension culture is usually used when culturing microalgae. In cases where such type of photobioreactor is applied, light must pass through a large water volume in order to activate the photosynthesis of the algae. The larger the diameter of the photobioreactor, the lower the light yield. While the cell density increases over time, the algae cells shade themselves and the productivity decreases significantly. At the same time, if the lighting is too intense, the algae cells are damaged. This is a central problem of suspension cultivation. In order to optimize light yields, photobioreactors were developed further.

In US 6 509 188 B1 for example, the authors describe a suspension based photobioreactor which generates increased growth rates by optimized use of lighting. The problem that light has to permeate the water volume, though, is still not solved hereby.

Through stirring, the suspension is constantly kept in motion in order to guarantee sufficient mixing. However, microalgae, which naturally live sessile (attached to surfaces), can only be cultivated swimming freely in a water column to a limited extent. Strong water movements destroy the cell walls of, e.g., many diatoms (often sessile), such as *Odontella sp.* so that the algae die.

The systems based on porous substrates as described by Podola et al. (Podola, Li, and Melkonian: "Porous Substrate Bioreactors: A Paradigm Shift in Microalgal Biotechnology?", Trends in Biotechnology, February 2017, Vol. 35, No. 2, 121-132) enable cultivation of microalgae separately from water. This is achieved through a two layer membrane. The algae cells do not grow swimming freely in a water column, but attached to a membrane and are only kept moist. Cultivating algae biomass in an immobilized biofilm attached to a membrane reduces the required water volume. Also, harmful shearing forces are avoided. Membrane-based photobioreactors reduce the water consumption for algae biomass production while also the gas exchange is improved.

It is known from the state of the art that for the production of *Odontella aurtia* a reduction of the production temperature has a positive impact on the EPA share in the algae cells (Pasquet et al. (2014): "Fatty acids profile and temperature in the cultured marine diatom Odontella aurita.", J Appl Phycol 26:2265-2271). A temperature reduction from 24 to 8 °C increases the EPA share of the total lipid composition to around 20-30%. At the same time though, the production rate decreases by 40%. To establish a commercially profitable production system, a high production rate at high temperature followed by the enrichment of EPA at low temperature is necessary. With current production systems the large water volume is a big challenge. It must be heated and cooled down, which is costly and energy intensive.

### Summary of the invention

It is an object of the invention to solve the above-mentioned problems of prior art systems and methods and to provide a method for producing at least one polyunsaturated fatty acid (PUFA) using microalgae with increased productivity and more efficient and less costly enrichment of unsaturated fatty acids.

The object is met by a method as initially specified which is characterized in that the microalgae are immobilized on at least one porous substrate and their cultivation is employed on said substrate. That is, the invention concerns a cultivation process for microalgae immobilized on a porous substrate such as, e.g., a membrane, so as to efficiently produce unsaturated fatty acids. For example, the microalgae can be cultivated on a membrane based photobioreactor. This production procedure facilitates the regulation of light and temperature and thus enables an efficient enrichment of unsaturated fatty acids, e.g. EPA and/or DHA, in the algae cells. By immobilizing the algae cells on a membrane, higher growth rates are obtained. Moreover, less liquid (e.g., water) or nutrient containing medium is needed for cultivation. Cooling and heating the smaller liquid volume is more efficient and less costly. The algae cells grow as a biofilm attached to the porous substrate and freely exposed to the ambient air. In combination with the large cultivation surface this enables a facilitated cooling of the biofilm through ambient air. Due to the separation of algae cells and liquid (water) or culture medium, a constant and effective disinfection of the water/medium is possible. In this way, the liquid volume must be exchanged less often and the temperature can be kept stable for a longer period of time. Due to the immobilization of the algae cells on the porous substrate and thus their perfect exposition to ambient light, the lighting of the algae cells is much more efficient. The light, which is necessary in order to stimulate photosynthesis, must not go through water columns anymore and can directly fall onto almost all algae cells.

The algae cells get use to the intensified light supply such that the cells do not get damaged. At the same time, the direct lighting of the algae cells facilitates the applied excessive light supply. This induces an intended and controlled stress reaction and therefore increases the enrichment of unsaturated fatty acids, for example, EPA and/or DHA.

Ambient light and/or artificial light can be provided to the algae cells in order to enable them to perform photosynthesis. According to an advantageous embodiment of the invention, if artificial light is used, the intensity of the light can be adjusted to a value between 200 and 1.000 µmol/m²/s, in particular between 400 and 800 µmol/m²/s. Alternatively, the light may be provided in the form of light pulses with an intensity between 20 to 2.000 µmol/m²/s, wherein each light pulse has a duration between 0.5 sec and 10 minutes, in particular between 1 sec and 1 min. In any case, lighting of the algae cells should be adjusted such that a controlled stress reaction and therefore increased production and enrichment of unsaturated fatty acids are induced in the cells.

Enrichment of unsaturated fatty acids can be further enhanced if the intensity of the light within a wavelengths range between 430 and 490 nm is increased compared to the intensity of the light of the other wavelengths. That is, it is beneficial if the specific wavelengths band between 430 and 490 nm (blue color) dominates the spectrum of the light used to illuminate the microalgae during cultivation. In an advantageous embodiment of the invention the percentage of blue light in the spectrum of the light used for cultivation is adjusted to about 70-80 %.

For example, in order to achieve increased enrichment of unsaturated fatty acids during cultivation, the method according to the invention may comprise:
- a general increase of the light intensity within the wavelengths band between 430 and 680 nm up to 1,000 µmol/m²/s;
- a change of the spectrum of the light towards blue color (430-490 nm) up to about 80% blue; and/or
- application of light pulses, each having an intensity up to about 2,000 µmol/m²/s and a duration between 0.5 seconds and 10 minutes.

According to another advantageous embodiment of the invention the cultivation is performed at a temperature between 5°C and 25°C, in particular between 6°C and 16°C. For example, the temperature may be kept constant at 8°C. This significant reduction of the temperature further increases the intended and controlled stress reaction and thus ensures additional production and enrichment of unsaturated fatty acids, in particular EPA and/or DHA.

It is advantageous and further optimizes the method according to the invention if the cultivation is performed under at least one of the following additional conditions:
- the light has a wavelength between 300 and 780 nm, or between 380 and 490 nm, or between 430 and 490 nm, or between 430 and 680 nm, or between 630 and 680 nm;
- the cultivation is performed at a pH value between 7.5 and 8.5, in particular between 7.9 and 8.2;
- the cultivation is performed at a CO₂ concentration between 0.04 % and 10 %, in particular between 1 and 3 %;
- the cultivation is performed at a humidity between 35 % and 90 %, in particular between 60 and 80 %.

In an initial step, before the microalgae are cultivated for producing the polyunsaturated fatty acids, the microalgae may be expanded for maximizing biomass. This expansion of the algae cells is to be performed under conditions optimized for cell growth and proliferation. For example, the expansion can be performed in a suspension culture reactor or a membrane bioreactor. In an advantageous embodiment of the invention the expansion of the microalgae cells is performed on the same porous substrate, i.e. using the same bioreactor, as used for producing the polyunsaturated fatty acids.

In order to optimize the conditions for maximum cell growth and proliferation, during the expansion, the intensity of the light should be adjusted to a value of about 300 µmol/m²/s. However, in an even more advantageous approach, during the first days of the expansion (e.g. day 0-2), the light intensity may be adjusted to a value between 40 and 200 µmol/m²/s, and during the following days (e.g. from day 2 until the harvest of the biomass), the light intensity may be increased to a least 80 but not more than 800 µmol/m²/s, in particular about 300 µmol/m²/s. The light intensity can be evenly distributed over the whole range of wavelengths used for expansion. By these measures, production of algae biomass can be further enhanced.

It is advantageous and further increases the growth rate of the microalgae cells if the expansion is performed under at least one of the following additional conditions:
- the light has a wavelength between 300 and 780 nm, or between 380 and 490 nm, or between 430 and 490 nm, or between 430 and 680 nm, or between 630 and 680 nm;
- the expansion is performed at a temperature between 15°C and 28°C, in particular between 23°C and 25°C;
- the expansion is performed at a pH value between 7.5 and 8.5, in particular between 7.9 and 8.2;
- the expansion is performed at a CO₂ concentration between 0.04 % and 10 %, in particular between 1 and 3 %;
- the expansion is performed at a humidity between 35 % and 90 %, in particular between 60 and 80 %.

According to a further advantageous embodiment of the invention a flow rate of a liquid (e.g., water) or medium comprising the nutrient through the membrane is set to a speed between 10 ml/m²/min and 250 ml/m²/min, in particular between 30 ml/m²/min and 70 ml/m²/min. For example, the flow rate may be set to a speed of about 50 ml/m²/min. By these measures, production of algae biomass can be further enhanced.

In another advantageous embodiment of the invention the nutrient comprises, in order to further enhance biomass production, at least one carbon source selected from the group consisting of starch, glucose, saccharose, xylose, arabinose, lactose, lactate, cellulose, and acetate.

Basically, the porous substrate (i.e. a first substrate and/or any further substrate) may comprise at least one first surface for carrying the microalgae cells, wherein the substrate is disposed such that at least the first surface is exposed to ambient air. The porous substrate may further comprise at least one second surface which is at least partially in contact with the liquid (water) or medium comprising the nutrient(s), wherein the liquid or medium can pass through the porous substrate so as to provide the first surface and thus the algae cells with the nutrient(s).

In an advantageous embodiment the porous substrate itself (i.e. a first substrate and/or any further substrate) may have a sandwich-like configuration wherein a second surface and a third surface of this substrate are arranged such that they delimit a fluid path through which the liquid (water) or medium comprising the nutrient(s) can flow and get in contact with the second surface. In such configuration, the liquid or medium can pass through the second surface to the first surface so as to provide the first surface and thus the algae cells with the nutrient(s).

In a bioreactor for performing the method according to the invention two or more porous substrates may be provided, for example, a first substrate and a second substrate. Providing two or more substrates allows for easy expansion of the area available for cultivation of the microalgae cells. Thus, upscaling of the bioreactor's capacity can be easily achieved by multiplying the number of substrates. In such embodiments the first substrate and the second substrate can be arranged such that they delimit a fluid path where the liquid or medium can flow between the two substrates and wet their respective second surfaces. In particular, two flat, sheet- or plate-like substrates may be arranged parallel to each other so as to build a kind of channel representing the fluid path. In this advantageous sandwich-like configuration the liquid or medium can flow between the two substrates without the need to provide additional elements for guiding the fluid flow.

At least one additional element, such as a stabilizing or mounting element, can be disposed between two neighboring substrates and/or within the fluid path.

In order to ensure even and complete wetting of the second surface(s) of the substrate(s), the fluid path may comprise at least one porous material being suitable to evenly spread the liquid or medium within the fluid path. Preferably, the porous material may comprise a spacer fabric or the like. For example, the porous material, such as a spacer fabric or the like, may be part of the substrate and can be disposed in the fluid path between two sheets or membranes, i.e. the second and the third surface.

According to another advantageous embodiment of the invention the porous substrate comprises at least one microporous membrane. The microporous membrane may comprise or consist of polyethersulfone (PES). Such membranes are permeable to fluid but impermeable to algae cells and therefore well-suited for cultivating and expanding microalgae in the method according to the invention. In order to further enhance the affinity of microalgae to specific substrates (e.g., PES membranes), the first surface of the porous substrate may comprise an immobilizing material for bearing at least one biofilm comprising the microalgae. The immobilizing material can be a compound which modifies the substrate's surface or a substance which serves as a link between the surface and the cells.

For example, the polyunsaturated fatty acid (PUFA) is at least one of eicosapentaenoic acid (EPA, timnodonic acid) and docosahexaenoic acid (DHA, cervonic acid). However, other polyunsaturated fatty acids can be produced by the method according to the invention as well:
**Omega-3 fatty acids:** Hexadecatrienoic acid (HTA), Alpha-linolenic acid (ALA), Stearidonic acid (SDA), Eicosatrienoic acid (ETE), Eicosatetraenoic acid (ETA), Heneicosapentaenoic acid (HPA), Docosapentaenoic acid (DPA, Clupanodonic acid), Tetracosapentaenoic acid, and Tetracosahexaenoic acid (Nisinic acid).
**Omega-6 fatty acids:** Linoleic acid (LA), Gamma-linolenic acid (GLA), Eicosadienoic acid, Dihomo-gamma-linolenic acid (DGLA), Arachidonic acid (AA), Docosadienoic acid, Adrenic acid (AdA), Docosapentaenoic acid (Osbond acid), Tetracosatetraenoic acid, and Tetracosapentaenoic acid.

For producing such PUFAs in the method according to the invention microalgae selected from the group consisting of *Schizochytrium sp.* (Labyrinthulea, Thraustochytrida), *Crypthecodinium sp.* (Dinophyceae, Peridiniales), *Phaeodactylum sp.* (Bacilariophyceae, Naviculales), *Haslea* sp. (Bacilariophyceae, Naviculales), Symbiodinium sp. (Dinophyceae, Suessiales) and especially *Odontella sp.* (Bacilariophyceae, Triceratiales) are well-suited.

According to an advantageous embodiment of the invention eicosapentaenoic acid (EPA) and docosahexaenoic acid (DHA) are produced using *Odontella sp.* Cells, in particular *Odontella aurita.*

Besides culture medium and other nutrient-containing solutions waste or process water from other processes such as aquaculture can also be used as nutrient source for culturing the microalgae. Aquaculture facilities produce aquatic organisms under controlled conditions. Nutrients accumulate in the water through the metabolic products of animals as well as feed residue. As a consequence, the water quality decreases, which limits the productivity of the culture. To solve this problem, closed recirculating systems ("RAS systems") have been established. In these systems, the water constantly passes biological and mechanical filtration processes and is afterwards fed back into the animal pond. This process accumulates nitrogen in the facility which must be constantly washed out through the addition of fresh water. Nitrogen compounds, however, serve as excellent nutrient source for phototrophic organisms such as microalgae.

The invention is further described in detail with reference to the following figures and examples.

### Brief description of the figures

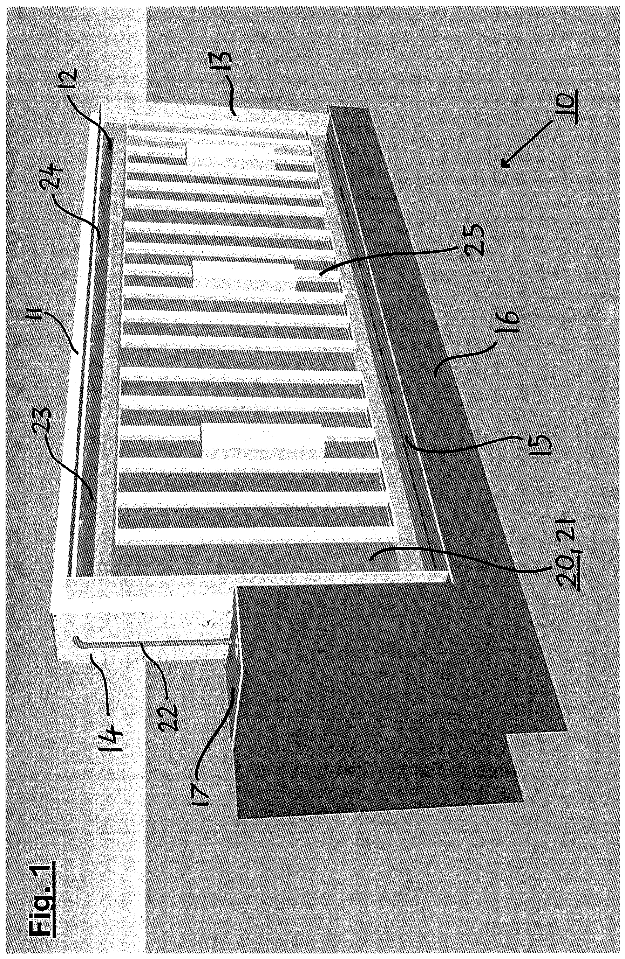

**Figure 1** shows a perspective representation of an embodiment of a bioreactor for performing the method according to the invention.

### Detailed description of exemplary embodiments of the invention

Basically, the invention relates to a production process of unsaturated fatty acids, in particular EPA from the microalgae species Odontella (Phylum: Ochrophyta; Class: Bacillariophyceae; Order: Triceratiales). The microalgae are cultivated on a membrane based photobioreactor, which simplifies the temperature and light control significantly. So that the concentration of unsaturated fatty acids in the algae cells increase.

**Figure** 1 shows an embodiment of a bioreactor 10 for conducting the method according to the invention. The bioreactor 10 is a photobioreactor with an exemplary size of 4000 x 2000 x 400 mm. The component parts of the bioreactor 10 are preferably at least in part made of food-safe plastics. The bioreactor 10 comprises at its top a cross brace 11 and a first substrate suspension 12, the first substrate suspension 12 being disposed in parallel to the cross brace 11 and attached to it. A second substrate suspension (not visible) is attached to the cross brace 11, wherein this second substrate suspension is disposed in parallel to the first substrate suspension 12. The bioreactor 10 further comprises two side supports 13, 14 which connect the cross brace 11 to a cover plate 15 at the bottom of the bioreactor 10. A container 16 (e.g., a plastic tank) is welded or glued to the cover plate 15 (e.g., a plastic plate). Two connecting elements (not visible) forming a drainage channel (not visible) are glued or welded to the cover plate 15 at the top of the container 16. Furthermore, the bioreactor 10 comprises a small fluid processing unit 17 which may include, for example, a membrane pump, a pressure reducer, a manometer, a filter candle with a pore size of 1 µm, and a UV clarifier. Additionally, control sensors, which may include detectors for measuring CO2, temperature, light intensity (µmol/s/m2), pH, conductivity, filling level, leakage, and pressure, as well as other control devices such as pump control (on/off), fan control (on/off/control), LED control (on/off/control), and image recognition of the cultivation surfaces, can be installed in the bioreactor 10.

A first substrate 20 is attached to the substrate suspension 12, at the opposite end to a first connecting element at the drainage channel, and laterally to the side supports 13, 14. The substrate 20 is designed to carry microalgae at a first surface 21 (outer surface) which is exposed to the ambient air. In this embodiment the substrate 20 comprises a microporous membrane having a first surface 21 which is at least partially exposed to ambient air and, at the opposite side of the substrate 20, a second surface (not visible) which is in contact with a liquid (e.g., water), a culture medium or another nutrient solution, wherein the membrane is permeable to the liquid or medium so that the liquid or medium can pass from the second surface through the membrane to the first surface 21. Accordingly, microalgae that are immobilized on the first surface 21 can utilize the nutrient(s) provided with the liquid or medium. In an advantageous embodiment of the invention the substrate 20 may comprise two membranes that are arranged parallel to each other so as to build a channel between them, which represents a fluid path where the liquid or medium can flow between the two membranes. A porous material can be provided in the fluid path in order to ensure even distribution of the liquid or medium. Thus, such sandwich-like configuration comprises three layers, a porous material (e.g., a spacer fabric material) in the middle and two fine-pored membranes (e.g., made of polyethersulfone with pore sizes of 0.2 - 20 µm) on the outer faces. The three layers can be attached to each other by a thermoplastic adhesive.

The surface area available for cultivation of the microalgae cells can be easily expanded by the provision of two or more porous substrates, for example, a first substrate and a second substrate. Accordingly, upscaling of the bioreactor's 10 capacity can be easily achieved by multiplying the number of substrates. For example, a second substrate (not visible) can be attached to the second substrate suspension, at the opposite end to a second connecting element at the drainage channel, and laterally to the side supports 13, 14
A pump (e.g., a membrane pump) pumps the liquid or medium from the container 16 through the fluid processing unit 17 and tubing 22 into a fluid distribution bar 23. The fluid distribution bar 23 comprises droppers 24 attached to it and distributes the liquid or medium evenly over the second surface of the substrate 20 or a porous material between two substrates. The liquid or medium spreads over the second surface or the middle layer (porous material; e.g. a spacer fabric material) and thus provides the first surface(s) 21 of the substrate(s) (fine pored membranes) with the liquid or medium. The microalgae cells are applied to the first surface 21 and immobilized there. The liquid or medium comprising the nutrient(s) passes through the pores of the fine pored membrane to the biofilm consisting of algae cells and supplies the cells with water and nutrients.

The liquid or medium comprising the nutrient(s) can flow from the fluid distribution bar 23 through the fluid path along the second surfaces of the substrate(s) and the drainage channel into the container 16 where it is collected until it is recirculated into the fluid processing unit 17. By the provision of a fluid circuit within which the liquid or medium can circulate through a fluid processing unit and the fluid path along the second surface of the first substrate, the amount of the liquid or medium (e.g., water or culture medium) necessary for cultivation of the phototrophic organisms can be further reduced. The fluid processing unit 17 may further comprise filter element(s), cooling and/or heating device(s), ventilation equipment, heat exchanging device(s), pressure reducer(s), at least one pump, and devices for measuring the quality of the fluid, such as pH meter, conductivity meter, thermometer, and manometer. However, such equipment can, additionally or alternatively, also be disposed outside the fluid processing unit 17 and/or the bioreactor 10. In any case, adjusting the temperature of the circulating liquid or medium can be easily accomplished and controlled in such a configuration since the amount of liquid or medium is relatively low and the algae layer attached to the porous substrate is thin. For example, the temperature of the liquid or medium within the bioreactor 10 can be adjusted to a value between 5 °C and 25 °C, in particular between 6 and 16 °C. This relatively low temperature further increases the intended and controlled stress reaction and thus ensures even more efficient production and enrichment of unsaturated fatty acids in the algae cells.

The bioreactor 10 is further equipped with artificial light sources 25 (LEDs, metal halide lamps) but can also or alternatively be operated with sunlight. If artificial light is used, the intensity of the light can be adjusted to a value between 200 and 1.000 µmol/m²/s, in particular between 400 and 800 µmol/m²/s. In order to further increase production and enrichment of unsaturated fatty acids in the algae cells, the light may be provided in the form of light pulses with an intensity between 20 to 2.000 µmol/m²/s, wherein each light pulse has a duration between 0.5 sec and 10 minutes, in particular between 1 sec and 1 min. Such lighting causes a controlled stress reaction in the cells and leads to increased production and enrichment of unsaturated fatty acids.

Until harvest, the algae cells are cultivated on the fine pored membrane and can then be harvested with mechanical forces such as scraping or ultrasound. A chemical treatment with surfactant agents and/or organic solvents is also possible. Further harvesting procedures are possible:
A) The algae biomass is harvested together with the fine pored membrane;
B) Detached biomass from the membrane is collected in flowing medium;
C) Detached and dried biomass from the porous carrier material is collected.

After harvesting, the wet biomass can directly be used to extract and isolate the unsaturated fatty acids. Moreover, the biomass can be dried and afterwards be processed as powder or pellets. The resulting powder as well as the pellets can be also used to extract and isolate unsaturated fatty acids therefrom.

In the following an exemplary production process is described in detail, with which maximization of biomass as well as enrichment of unsaturated fatty acids in a membrane-based bioreactor or exclusively the enrichment of unsaturated fatty acids in a membrane-based bioreactor can be ensured.

Briefly, the production process comprises the following steps:
Supply of *Odontella sp.* biomass as inoculum to start-up a membrane-based photobioreactor, for example, the bioreactor 10 according to Figure 1;
Applying and immobilizing of the biomass on the membrane surface; Expansion of *Odontella sp.* to maximize biomass;
Cultivation of *Odontella sp.* for an optimized enrichment of EPA and DHA;
Harvesting of the biomass; and
Isolation of the enriched unsaturated fatty acids, in particular EPA and DHA.

The biomass of *Odontella sp.* as inoculum for the photobioreactor can be provided by a previous production cycle from the same and/or another and/or multiple other bioreactors, from one and/or multiple suspension starter cultures, by a previous production cycle from one and/or multiple suspension reactors, or by a previous production cycle from one and/or multiple fermentation reactors. One can, but is not limited to, attach and immobilize the biomass on the membrane surface with the help of spray, filtration or brush procedures.

The described production procedure of *Odontella sp.* includes two cultivation steps. First, the algae *Odontella sp.* is expanded under perfect conditions in order to maximize biomass. Subsequently, poly unsaturated fatty acids (PUFA) such as eicosapentaenoic acid (EPA) and docosahexaenoic acid (DHA) are enriched in a second optimized membrane based cultivation step. The expansion step for the biomass maximization of the algae cells on the membrane can take between 5 and 30 days. In the best case, the expansion takes 10 days. During the first days of the expansion step (e.g. day 0-2), the light intensity should be adjusted to at least 40 and at most 200 µmol/m²/s. In the following days (e.g. from day 2 until the harvest of the biomass), the light intensity should be increased to a least 80 but not more than 800 µmol/m²/s. In the best case, from day 2 until the harvest of the biomass, the light intensity is approximately set to 300 µmol/m²/s. Also conceivable is an approach where from day 0 onwards the algae cells are constantly lit with an intensity of about 300 µmol/m²/s. The wavelength of the light for the biomass concentration of *Odontella sp.* should be selected between 300 and 780 nm or at least cover the specific wavelength range between 430 and 490 nm as well as between 630 and 680 nm. This enables an optimized photosynthetic activity of the algae cells and thus a maximization of biomass production.

In order to further maximize the biomass of *Odontella sp.,* the process temperature should be adjusted to at least 15°C but not more than 28°C during the expansion step. Ideally, the temperature lies between 23°C and 25 °C. The temperature can be regulated through a heat exchanger in the container (tank) of the bioreactor or, better, through a direct inflow into the membrane (e.g., by a heating/cooling device within a fluid processing unit of the bioreactor). Furthermore, the temperature can be varied through the ambient temperature. During the expansion step (maximizing the biomass of *Odontella sp*.), the pH-value should be adjusted to at least 7.5 and not more than 8.5. In the optimal case, the pH-level lies between 7.9 and 8.2.

For providing the algae cells with nutrient(s) during the expansion phase, for example, a culture medium may be used (e.g., F/2 + Si of Guillard and Ryther, 1962). In order to maximize biomass of *Odontella sp.* the medium should flow through the membrane at a speed of at least 10 ml/m²/min, but not more than 250 ml/m²/min, during the expansion phase. In the best case, the flow rate is adjusted to a speed between 30 ml/m²/min and 70 ml/m²/min. In the optimal case, it is set to about 50 ml/m²/min. Also, a mixotrophic expansion approach is conceivable. In this case, a carbon source such as starch, glucose, saccharose, xylose, arabinose, lactose, lactate, cellulose and/or acetate can be added to the culture medium.

During the expansion step, in order to maximize biomass of *Odontella sp.,* the CO₂ concentration should be adjusted to at least 0.04 % and not more than 10 %. In the optimal case, it is set to a concentration between 1 % and 3 %. In order to maximize biomass of *Odontella sp.* during the expansion step, the humidity within the bioreactor should be adjusted to a concentration of at least 35 % and not more than 90 %. In the optimal case, it is set to a concentration between 60 % and 80 %.

The cultivation step to enrich unsaturated fatty acids, in particular EPA and/or DHA, in the algae *Odontella sp.* can take between 1 and 10 days. This step is performed after the biomass production ("expansion") through a previous production cycle from the same and/or another and/or multiple other membrane reactors, from one and/or multiple suspension starter cultures, through a previous production cycle from one and/or multiple suspension reactors, or through a previous production cycle from one and/or multiple fermentation reactors has been completed.

In case of biomass maximization through a previous production cycle from the same and/or another and/or multiple other membrane reactors, from one and/or multiple suspension starter cultures, through a previous production cycle from one and/or multiple suspension reactors, or through a previous production cycle from one and/or multiple fermentation reactors, the algae cells must be immobilized on the membrane. The biomass can be attached and immobilized on the membrane surface with, exemplary but not necessarily, help of a spray, filtration, or brush procedure.

During the cultivation phase to enrich the unsaturated fatty acids, in particular EPA and/or DHA, the light intensity should be adjusted to at least 200 and at most 1.000 µmol/m²/s. In the best case, the intensity is set to a value between 400 and 800 µmol/m²/s. The increased light intensity causes a stress reaction of the algae cells. Through light pulses with an intensity of 20 to 2.000 µmol/m²/s at a frequency of 0.5 sec to 10 minutes, better at a frequency of 1 sec to 1 min, the algae cells can also be stressed. The wavelength of the light used for the enrichment of the unsaturated fatty acids should be set between 300 and 780 nm or at least cover the specific light wavelengths between 430 and 490 nm as well as between 630 and 680 nm. In the best case, enrichment of unsaturated fatty acids can be increased if the intensity of the light within a wavelengths band between 430 and 490 nm is increased compared to the intensity of the light of the other wavelengths, i.e. if blue color dominates the spectrum of the light used to illuminate the microalgae during cultivation.

During the cultivation phase to enrich the unsaturated fatty acids, in particular EPA and/or DHA, the temperature should be adjusted to at least 5°C and not more than 25°C. In the best case, it is set to a value between 6°C and 16°C. In the optimal case, the temperature is kept constant at 8°C. As the algae cells are separated from the liquid or culture medium by the porous substrate, a continuous sterilization of the liquid or culture medium through UV disinfection is possible. This way, the liquid or culture medium volume must be exchanged less often and the temperature in the reactor can be kept stable for a longer period of time. In combination with the large cultivation surfaces this facilitates the way of cooling the biofilm through ambient air. One can regulate the temperature through the heat exchanger inside the container of the bioreactor or better directly at the membrane inflow, for example, by means of suitable devices within a fluid processing unit of the bioreactor. Moreover, one can adjust the temperature through the ambient air.

During the cultivation phase to enrich the unsaturated fatty acids, in particular EPA and/or DHA, the pH value should be adjusted to at least 7.5 and not higher than 8.5. In the optimal case, it is set between pH 7.9 and pH 8.2. For providing the algae cells with nutrient(s) during the cultivation phase, for example, a culture medium may be used (e.g., F/2 + Si of Guillard and Ryther, 1962). During the cultivation phase to enrich the unsaturated fatty acids, in particular EPA and/or DHA, the medium should flow through the membrane at a speed of at least 10 ml/m²/min but not more than 250 ml/m²/min. In the best case, the flow rate is set to a speed between 30 ml/m²/min and 70 ml/m²/min. In the optimal case, it is set to a speed of about 50 ml/m²/min. Also, a mixotrophic cultivation approach is conceivable. In this case, a carbon source such as starch, glucose, saccharose, xylose, arabinose, lactose, lactate, cellulose or acetate can be added to the culture medium.

During the cultivation phase to enrich the unsaturated fatty acids, in particular EPA and/or DHA, the CO₂ concentration should be adjusted to a value of at least 0.04 % and not more than 10 %. In the optimal case, the concentration is set between 1 % and 3 %. During the cultivation phase to enrich the unsaturated fatty acids, in particular EPA and/or DHA, the humidity within the bioreactor should be adjusted to a concentration of at least 35 % and not more than 90 %. In the optimal case, it is set to a concentration between 60 % and 80 %.

After cultivation, the algae biomass can be harvested using processes such as scraping, blowing, washing, chemical treatment, drying and mechanical harvesting, drying and harvesting the cells together with the membrane, or a combination of these processes.

Suitable methods to isolate the unsaturated fatty acids, in particular EPA and/or DHA, from the algae cells are known to a person skilled in the art. For example, extraction and isolation of the fatty acids can be achieved by CO₂ extraction. The extracted unsaturated fatty acids, in particular EPA and/or DHA, can then be used as food supplement, feed supplement or as an ingredient in therapeutic products.

## Claims

1. Method for producing at least one polyunsaturated fatty acid using microalgae, wherein the microalgae are cultivated by exposing the microalgae to light and providing them with at least one nutrient, **characterized in that** the microalgae are immobilized on at least one porous substrate and their cultivation is employed on said substrate.

2. Method according to claim 1, wherein the intensity of the light is adjusted to a value between 200 and 1.000 µmol/m²/s, in particular between 400 and 800 µmol/m²/s.

3. Method according to claim 1, wherein the light is provided in the form of light pulses with an intensity between 20 to 2.000 µmol/m²/s, wherein each light pulse has a duration between 0.5 sec and 10 minutes, in particular between 1 sec and 1 min.

4. Method according to any one of claims 1 to 3, wherein the intensity of the light within a wavelengths range between 430 and 490 nm is increased compared to the intensity of the light of the other wavelengths.

5. Method according to any one of claims 1 to 4, wherein the cultivation is performed at a temperature between 5°C and 25°C, in particular between 6°C and 16°C.

6. Method according to any one of claims 1 to 5, wherein the cultivation is performed under at least one of the following conditions:
- the light has a wavelength between 300 and 780 nm, or between 380 and 490 nm, or between 430 and 490 nm, or between 430 and 680 nm, or between 630 and 680 nm;
- the cultivation is performed at a pH value between 7.5 and 8.5, in particular between 7.9 and 8.2;
- the cultivation is performed at a CO₂ concentration between 0.04 % and 10 %, in particular between 1 % and 3 %;
- the cultivation is performed at a humidity between 35 % and 90 %, in particular between 60 % and 80 %.

7. Method according to any one of claims 1 to 6, wherein in an initial step, before the microalgae are cultivated for producing the polyunsaturated fatty acids, the microalgae are expanded for maximizing biomass.

8. Method according to claim 7, wherein the expansion is performed in a suspension culture reactor or a membrane bioreactor.

9. Method according to claim 7 or 8, wherein during the expansion the intensity of the light is adjusted to a value of about 300 µmol/m²/s, or wherein during the first days of the expansion (e.g. day 0-2), the light intensity is adjusted to a value between 40 and 200 µmol/m²/s, and during the following days (e.g. from day 2 until the harvest of the biomass), the light intensity is increased to a least 80 but not more than 800 µmol/m²/s, in particular about 300 µmol/m²/s.

10. Method according to any one of claims 7 to 9, wherein the expansion is performed under at least one of the following conditions:
- the light has a wavelength between 300 and 780 nm, or between 380 and 490 nm, or between 430 and 490 nm, or between 430 and 680 nm, or between 630 and 680 nm;
- the expansion is performed at a temperature between 15°C and 28°C, in particular between 23°C and 25°C;
- the expansion is performed at a pH value between 7.5 and 8.5, in particular between 7.9 and 8.2;
- the expansion is performed at a CO₂ concentration between 0.04 % and 10 %, in particular between 1 % and 3 %;
- the expansion is performed at a humidity between 35 % and 90 %, in particular between 60 % and 80 %.

11. Method according to any one of claims 7 to 10, wherein a flow rate of a liquid or medium comprising the nutrient through the membrane is set to a speed between 10 ml/m²/min and 250 ml/m²/min, in particular between 30 ml/m²/min and 70 ml/m²/min.

12. Method according to any one of claims 7 to 11, wherein the nutrient comprises at least one carbon source selected from the group consisting of starch, glucose, saccharose, xylose, arabinose, lactose, lactate, cellulose, and acetate.

13. Method according to any one of claims 1 to 12, wherein the porous substrate comprises at least one microporous membrane.

14. Method according to any one of claims 1 to 13, wherein the polyunsaturated fatty acid is at least one of eicosapentaenoic acid and docosahexaenoic acid, or wherein the polyunsaturated fatty acid is selected from the group consisting of hexadecatrienoic acid, alpha-linolenic acid, stearidonic acid, eicosatrienoic acid, eicosatetraenoic acid, heneicosapentaenoic acid, docosapentaenoic acid, tetracosapentaenoic acid, tetracosahexaenoic acid, linoleic acid, gamma-linolenic acid, eicosadienoic acid, dihomo-gamma-linolenic acid, arachidonic acid, docosadienoic acid, adrenic acid, docosapentaenoic acid, tetracosatetraenoic acid, and tetracosapentaenoic acid.

15. Method according to any one of claims 1 to 14, wherein the microalgae are selected from the group consisting of Schizochytrium sp. (Labyrinthulea, Thraustochytrida), Crypthecodinium sp. (Dinophyceae, Peridiniales), Phaeodactylum sp. (Bacilariophyceae, Naviculales), Odontella sp. (Bacilariophyceae, Triceratiales), Symbiodinium sp. (Dinophyceae, Suessiales) and Haslea sp. (Bacilariophyceae, Naviculales).
